Europäisches Patentamt

European Patent Office

Office européen des brevets

(11) Veröffentlichungsnummer: **0 198 212**
**B1**

(12) # EUROPÄISCHE PATENTSCHRIFT

(45) Veröffentlichungstag der Patentschrift:
**23.11.89**

(51) Int. Cl.⁴: **A61M 1/36**

(21) Anmeldenummer: **86103193.8**

(22) Anmeldetag: **10.03.86**

(54) Vorrichtung zur physiologisch-therapeutisch wirksamen optischen Bestrahlung körpereigenen Venenblutes.

(30) Priorität: **20.03.85 DD 274282**

(43) Veröffentlichungstag der Anmeldung:
**22.10.86 Patentblatt 86/43**

(45) Bekanntmachung des Hinweises auf die Patenterteilung:
**23.11.89 Patentblatt 89/47**

(84) Benannte Vertragsstaaten:
**AT CH DE FR GB LI**

(56) Entgegenhaltungen:
**DE-A- 2 926 523**
**DE-A- 2 927 260**
**DE-A- 3 239 417**
**DE-B- 957 877**
**DE-B- 1 215 867**
**US-A- 2 074 909**
**US-A- 4 464 166**

(73) Patentinhaber: **VEB Elektro- und Metallgeräte Ilmenau,
Weimarer Strasse 47 Postfach 150,
DDR-6300 Ilmenau(DD)**

(72) Erfinder: **Fisch, Joachim, Dr.-Ing.,
Corona-Schröter-Strasse 18, DDR-6300 Ilmenau(DD)**
Erfinder: **Kost, Hans-Richard, Dr. med., Oberer
Weinberg 11, DDR-6110 Hildburghausen(DD)**
Erfinder: **Riemann, Manfred, Prof. Dr. sc. techn.,
Corona-Schröter-Strasse 8, DDR-6300 Ilmenau(DD)**
Erfinder: **Sonnemann, Jörg,
Albert-Schweitzer-Strasse 24, DDR-3304 Gommern(DD)**
Erfinder: **Fisch, Gerda, Dipl.-Ing.,
Corona-Schröter-Strasse 18, DDR-6300 Ilmenau(DD)**

(74) Vertreter: **Zipse + Habersack, Kemnatenstrasse 49,
D-8000 München 19(DE)**

## Beschreibung

Die Erfindung betrifft eine Vorrichtung, mit deren Hilfe eine Bestrahlung körpereigenen Venenblutes das mit Natriumzitrat versetzt ist, überwiegend im UVA- und Blaulichtbereich möglich ist, nach dem Oberbegriff des Anspruchs 1. Solch eine Vorrichtung ist aus DE-A 2 926 523 bekannt. Vorrichtungen und Geräte zur speziellen Blutbehandlung werden seit Jahrzehnten vorgestellt und in der Medizin eingesetzt. Ihre Anwender sind von der physiologischen und/oder therapeutischen Wirkung der Blutbehandlung überzeugt.

Das Ziel aller bekannten technischen Lösungen besteht darin, das Blut so zu beeinflussen, daß im Körper vorhandene Regenerierungsvorgänge angeregt werden. Dabei sollen gestörte biologische Parameter im positiven Sinne verändert werden. Es sind Vorrichtungen und Geräte bekannt, bei denen Sauerstoff/Ozon-Gasgemische subkutan, intramuskulär, intravenös oder als Insufflation zur Anwendung kommen. Beispielsweise wird in der DD-A 14127 eine Vorrichtung zur Anreicherung ununterbrochen strömenden Blutes mit Sauerstoff vorgestellt. Weiterhin sind Vorrichtungen und Geräte bekannt, die in einem Quarzglasgefäß (Küvette oder Rohr) das Blut zum Zweck der Bestrahlung an einem UV-Strahler vorbeifließen lassen, wie es z. B. in den DE-C 10 65 140 und DE-C 10 71 291 sowie der DE-A 29 26 523 beschrieben ist. Es sind auch seit langem Einrichtungen bekannt, bei denen das Blut mittels Sauerstoff aufgeschäumt und dabei einer Ultraviolettbestrahlung unterzogen wird. Beispielsweise sind in der DE-C 957 877 und der DE-A 12 15 867 derartige Geräte zum Behandeln von Blut, Blutplasma o. dgl. mit Sauerstoff und gegebenenfalls mit ultravioletter Strahlung beschrieben. Die Verfahren wurden im Laufe der Zeit verbessert und für die Probanden wirksamer gemacht.

In den US-A 4 321 919 und US-A 4 464 166 ist eine Methode beschrieben, bei welcher körpereigenes Blut des Probanden durch eine Pumpe, eine Bestrahlungskammer und eine Zentrifuge wieder in den Körper zurückgelangt. Die Sterilitätsbedingungen werden dabei eingehalten, doch der technische Aufwand ist beträchtlich. Eine weitere Lösung wird in der DE-A 29 43 310 A 1 vorgestellt. Über ein mittels Punktion eingeführten Lichtleitkabel wird eine UV-Strahlung in das Gefäßsystem geführt und so eine biophysikalische Wirkung erzielt.

Alle bisher genannten Vorrichtungen und Geräte setzen einen beträchtlichen technischen und medizinischen Aufwand voraus. Ökonomische, personelle und Sterilitätsprobleme schränken den Einsatz stark ein. Verunreinigungen oder Verschleppung von Keimen im Blut sind bei vielen Verfahren nicht auszuschließen. Eine falsche Dosierung der Gase zur Aufschäumung des Blutes und der UV-Strahlung, z. B. durch menschliches Versagen, kann zu lebensgefährlichen Komplikationen wie Embolien, Gen- oder Zellschädigungen führen. Die Reinigung und Sterilisation der bekannten Vorrichtungen und Geräte ist sehr aufwendig. Bei den beschriebenen bekannten Verfahren zur Blutbestrahlung werden in der Regel UVC-Strahler eingesetzt. Dem UVC-Bereich der UV-Strahlung wird bisher vor allem die therapeutische Wirkung zugesprochen, schädliche Auswirkungen der kurzwelligen Ultraviolettbestrahlung auf Zell- und Gewebeeigenschaften sind jedoch nicht auszuschließen.

Demzufolge soll die Erfindung eine Vorrichtung schaffen, die

– gefahrloser für den Probanden wird und ausschließlich optische Strahlung, überwiegend im UVA- und Blaulichtbereich zur Anwendung bringt,
– in hygienischer Hinsicht wesentliche Verbesserungen ermöglicht und die Einhaltung der gesetzlichen Bestimmungen garantiert,
– schutztechnisch wesentlich verbessert ist und sowohl mit Netzspannung als auch mit Niederspannung betrieben werden kann,
– klein, leicht, netzunabhängig und damit mobil ist,
– billig in der Herstellung ist und eine hohe Servicefreundlichkeit aufweist.

Bei der Behandlung verschiedenartiger Erkrankungen ist es erforderlich, gestörte Atmungsvorgänge und Defizite in der Energieversorgung der Zellen, Störungen im Zusammenwirken von Zellen und Organen sowie Abweichungen der allgemeinen Stoffwechselparameter durch spezielle Behandlungsmaßnahmen (optische Bestrahlung des Blutes) in positiven Sinne zu verändern.

Derartige Maßnahmen bezwecken, Einfluß auf die gestörten Regulationsmechanismen zu nehmen, um die Selbstheilungsvorgänge des geschwächten Organismus zu aktivieren. Sie stellen eine weitere Therapieform gegenüber bisher bekannten Behandlungsverfahren dar. Für die optische Bestrahlung des Blutes werden in der Regel UVC-Strahler eingesetzt, obwohl auf mögliche schädliche Auswirkungen der UVC-Strahlung auf Zell- und Gewebeeigenschaften in der Literatur verwiesen wird.

Für eine Vorrichtung gemäß dem Oberbegriff des 1. Anspruches wird deshalb vorgeschlagen, nur langwellige, niederenergetische, überwiegend UVA- und Blaulichtenthaltende Strahlung (320 nm bis 600 nm) zum Einsatz zu bringen. Die physiologische und therapeutische Wirksamkeit ist nachgewiesen. Außerdem sind UVA- und Blaulichtstrahler technologisch wesentlich leichter herstellbar und billiger. Sie können beispielsweise als Nebenprodukt bei der Leuchtstofflampen-Serienfertigung, ohne oder mit speziellen Leuchtstoffen beschichtet, erhalten werden.

Erfindungsgemäß wird vorgeschlagen, einen Strahler, der überwiegend im UVA- und Blaulichtbereich strahlt, z.B. eine Xenonlampe, einzusetzen und mit geringer Leistung zu betreiben. Dabei wird mit geeigneten Mitteln zur Verhinderung der Blutgerinnung versetztes Venenblut in unterschiedlicher Durchsetzmenge bestrahlt. Das Venenblut wird direkt in aus PVC bestehenden medizinischen Einwegmaterialien in Form von PVC-Schläuchen behandelt, wobei die Strahlenexposition des Blutes durch Einstellen der Tropfzahl und der bestrahlten Schlauchlänge variierbar ist. Zur Verbesserung der Schutzgüte, zur Erhöhung des Wirkungsgrades des Strahlers, zur Veränderung der Intensität der Bestrahlung und zum Zwecke der Mobilität wird der Strahler durch ein elektronisches Vorschaltgerät mit regelbarer Hochfrequenzenergie versorgt. Ferner ist es möglich, die Erfindung mit Nieder- und/oder Netzspannung zu betreiben. Es wird ein ausreichend langer Schlauchanteil eines aus PVC bestehenden üblichen Bluttransfusionsgerätes, der für Strahlung mit Wellenlängen zwischen 320 nm und 600 nm durchlässig ist und keine toxischen Stoffe abgibt, in der geschlossenen Vorrichtung bestrahlt, wobei die der Strahlung ausgesetzte Schlauchlänge variiert werden kann.

Im folgenden wird die Erfindung anhand von Zeichnungen dargestellt, die lediglich eine mögliche Ausführungsform darstellen. Es zeigen:

Fig. 1 eine schematische Seitenansicht im Schnitt
Fig. 2 eine schematische Schnittdarstellung entlang der Linie II-II in Fig. 1

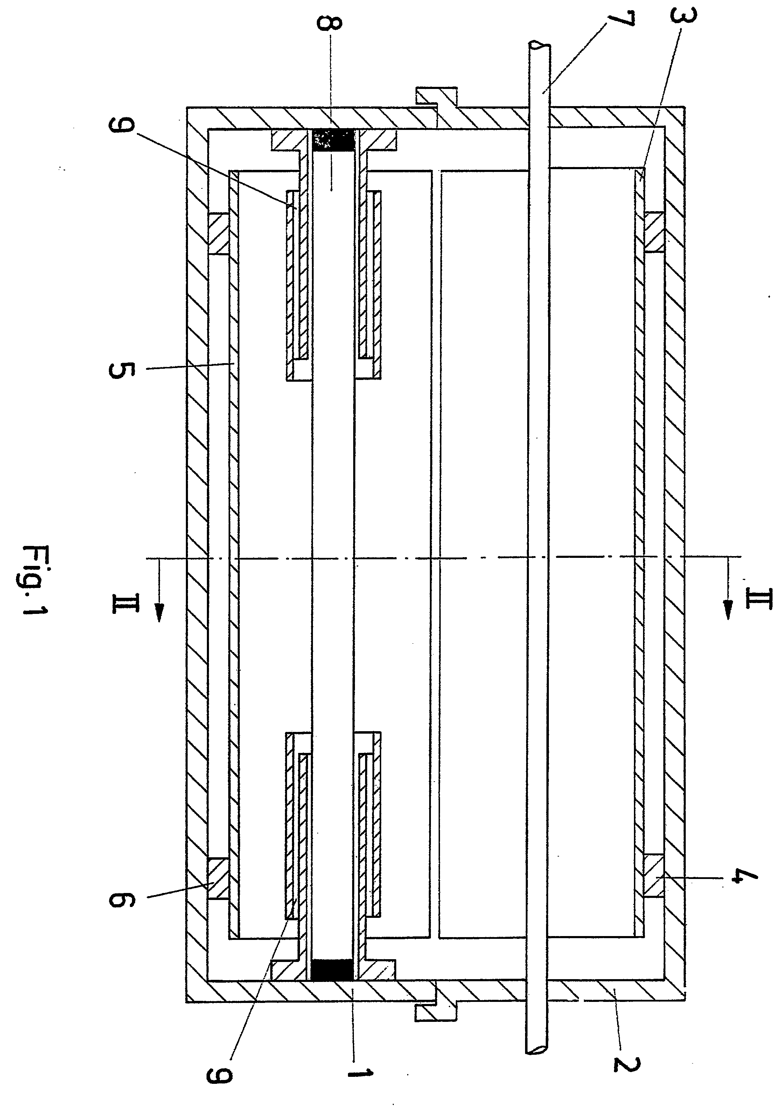

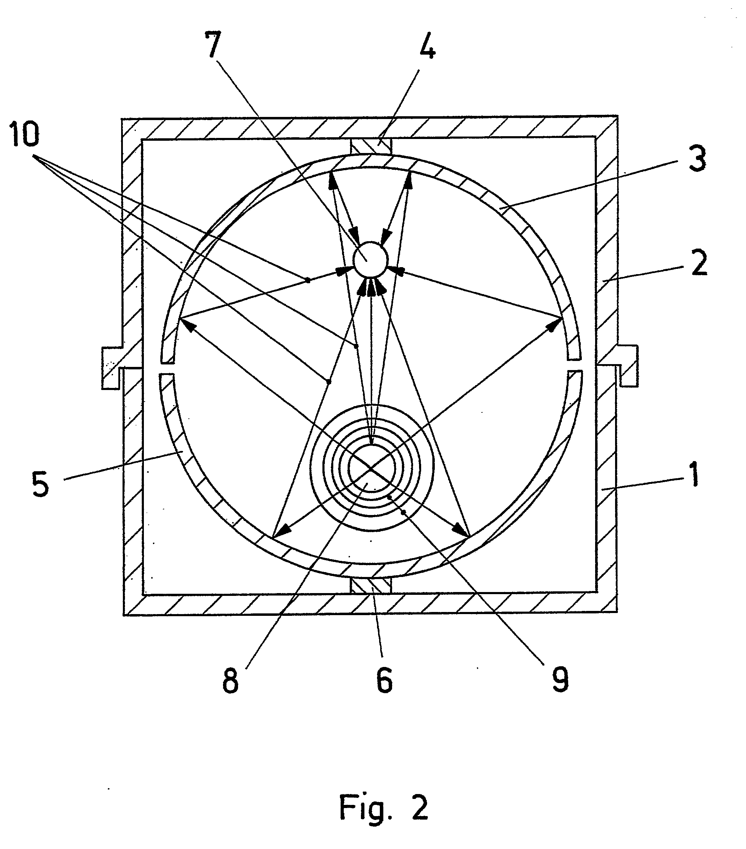

Die in den Figuren 1 und 2 dargestellte mögliche Ausführungsform der vorliegenden Erfindung zeigt ein Gehäuse 1, welches mit einem Deckel 2 verschließbar ist. Im Deckel 2 ist ein Reflektor 3 mit beliebigen nicht näher dargestellten Mitteln 4 befestigt. Ein weiterer gleichartiger Reflektor 5 ist im Gehäuse 1 mit geeigneten Mitteln 6 befestigt. Im Fokus des im Deckel 2 vorgesehenen Reflektors 3 ist ein PVC-Schlauch 7 angeordnet, der mittels geeigneter Befestigungselemente gehalten und durch Federn gespannt wird, damit er im wesentlichen auf der gesamten Länge des Reflektors möglichst exakt im Fokus liegt. Die Befestigungselemente und Federn sind zur Verbesserung der Übersichtlichkeit in der schematischen Darstellung weggelassen worden. Im Fokus des im Gehäuse 1 vorgesehenen Reflektors 5 ist ein Strahler 8 angeordnet, dessen Strahlung emittierende Länge mit Hilfe einer Teleskopanordnung 9, die rohrförmig um den Strahler 8 herum angeordnet ist, verändert werden kann. Dieser Strahler 8 wird mit Hilfe eines in seiner HF-Leistung variierbaren elektronischen Vorschaltgerätes (nicht dargestellt) betrieben, welches im Gehäuse, außen am Gehäuse 1, 2 oder auch als Zwischenschaltelement in der Zuleitung angeordnet sein kann.

Das Venenblut wird durch den PVC-Schlauch 7 durch den Deckel 2 des Gehäuses 1, 2 am Strahler 8 entlang geführt. Die Bestrahlung des Blutes im Schlauch 7 ist durch einzelne oder kombinierte Variation der Bestrahlungsstärke zwischen 1 mWcm$^{-2}$ und 10 mWcm$^{-2}$ sowie der Länge des bestrahlten Schlauchabschnitts von 1 cm bis 30 cm sowie der Durchflußmenge des bestrahlten Blutes von 20 Tropfen pro Minute bis 80 Tropfen pro Minute einstellbar. Die Länge des zu bestrahlenden Schlauchabschnitts kann mit Hilfe der Teleskopanordnung 9 eingestellt werden. Durch die Anordnung des PVC-Schlauches 7 und des Strahlers 8 im Fokus der jeweiligen Reflektoren 3, 5 wird erreicht, daß ein maximaler Wert der vom Strahler 8 abgegebenen Strahlung auf den PVC-Schlauch 7 gerichtet wird. Durch eine entsprechende Gestaltung des Gehäuses 1 und des Deckels 2 kann erreicht werden, daß ein gewünschter Abstand zwischen dem Strahler 8 und dem PVC-Schlauch 7 exakt vorhanden ist, wenn der Deckel 2 auf dem Gehäuse 1 sitzt. Die Reflektoren 3, 5 sind dann derart gestaltet, daß bei diesem Abstand die vom PVC-Schlauch 7 aufgenommene Strahlung den größten Wert erreicht. Die Reflektoren 3, 5 können auch so gestaltet sein, daß möglichst viele Strahlen 10 des Strahlers 8 auf den PVC-Schlauch 7 gerichtet werden. Die möglichen Wege der Strahlen 10 sind in Figur 2 schematisch dargestellt.

Als Vorschaltgerät wird zweckmäßig ein serienmäßiges Gerät verwendet, welches wahlweise mit Niederspannung ≤ 42 V oder mit Netzspannung 110/220 V betrieben werden kann. Die Vorrichtung ist in dieser Form transportabel und leicht zu handhaben und anzuwenden. Aus hygienischen Gründen wird der PVC-Schlauch 7 als Einwegmaterial verwendet und nach der Behandlung vernichtet.

Es ist natürlich auch möglich, die Teleskopanordnung 9 nur auf einer Seite anzuordnen, oder sie durch eine andere Abdeckung zu ersetzen, wie eine drehbare rohrförmige Blende mit einer wendelförmigen Endkante, die um den Strahler 8 herum angeordnet ist und durch Verdrehen die freie Abstrahllänge des Strahlers 8 verändert.

## Patentansprüche

1. Vorrichtung zur physiologisch und/oder therapeutisch optischen Bestrahlung körpereigenen Venenblutes, das mit Natriumzitrat versetzt ist, unter Verwendung von stabförmigen Strahlern (8) und einem Reflektorsystem (3, 5), dadurch gekennzeichnet, daß zumindest ein, überwiegend im Wellenlängenbereich 320 nm bis 600 nm emittierender Strahler (8) und ein Schlauch (7) aus medizinischem Einwegmaterial in einem Reflektorsysten (3, 5) so angeordnet sind, daß sie sich jeweils im Fokus des zugehörigen Reflektors (3, 5) befinden, daß das Reflektorsystem so angeordnet ist, daß der Schlauch (7) auf seinem gesamten Umfang durch direkte oder reflektierte Strahlen (10), die vom Strahler (8) emittieren, bestrahlt wird, daß weiterhin am Strahler (8) eine Abschattungsanordnung (9) vorgesehen ist, mit der die emittierende Länge des stabförmigen Strahlers (8) verändert werden kann, und daß die Bestrahlungsstärke durch Variation der vom an sich bekannten elektronischen Vorschaltgerät abgegebenen HF-Leistung variierbar ist.

2. Vorrichtung nach Anspruch 1, dadurch gekennzeichnet, daß als Strahler (8) eine Xenonlampe vorgesehen ist.

3. Vorrichtung nach Anspruch 1 und 2, dadurch gekennzeichnet, daß der zur Wirkung erforderliche Spektralbereich durch Aufbringung spezieller Leuchtstoffe auf den Strahler (8) realisiert ist.

4. Vorrichtung nach Anspruch 1 bis 3, dadurch gekennzeichnet, daß die Bestrahlung des Blutes im Schlauch (7) durch einzelne oder kombinierte Variation der Bestrahlungsstärke zwischen 1 mWcm$^{-2}$ und 10 mWcm$^{-2}$ sowie der Länge des bestrahlten Schlauchabschnittes von 1 cm bis 30 cm sowie der Durchflußmenge des bestrahlten Blutes von 20 Tropfen pro Minute bis 80 Tropfen pro Minute einstellbar ist.

**Claims**

1. Device for the physiological and/or therapeutical irradiation of autocorporal venous blood, which is citrated, using rod-shaped emitters (8) and a reflector system (3, 5), characterised in that at least one emitter (8), emitting mainly in the 320 nm to 600 nm wave range, and a tube (7) of disposable medical material are arranged in a reflector system (3, 5) in such a way that they are each in the focus of the associated reflector (3, 5), that the reflector system is arranged in such a way that the tube (7) is irradiated along its entire circumference by direct or reflected rays (10) emitted by the emitter (8), that, furthermore, the emitter (8) is provided with a shading arrangement (9), by means of which the emissive length of the rod-shaped emitter (8) can be varied, and that the irradiance can be varied by varying the RF power supplied by the electronic ballast unit known per se.

2. Device according to claim 1, characterised in that the emitter (8) is a xenon lamp.

3. Device according to claims 1 and 2, characterised in that the spectral range required for activation is obtained by coating the emitter (8) with specific luminescent materials.

4. Device according to claims 1 to 3, characterised in that the irradiation of the blood in the tube (7) can be adjusted by varying the irradiance between 1 mWcm$^{-2}$ and 10 mWcm$^{-2}$, varying the length of the irradiated tube section from 1 cm to 30 cm, and varying the irradiated blood flow rate from 20 drops per minute to 80 drops per minute, either individually or in combination.

**Revendications**

1. Dispositif pour l'irradiation optique de sang veineux auto-corporel efficace physiologiquement et thérapeutiquement, lequel sang est mélangé avec du citrate de sodium par utilisation d'irradiateurs tubulaires (8) et par un système de réflecteur (3, 5), caractérisé en ce qu'au moins un irradiateur (8) émettant principalement dans la gamme d'onde 320 nm à 600 nm et un tuyau (7) en matériau pour usage médical sont agencés dans un système de réflecteur de telle manière qu'il se trouve respectivement au foyer des réflecteurs (3, 5) correspondants, que le système de réflecteur est ainsi agencé que le tuyau (7) est irradié sur l'ensemble de son contour par des rayons (10) directs ou réfléchis qui sont émis par les irradiateurs (8), qu'en outre il est prévu au voisinage de l'émetteur (8) un système atténuateur avec lequel les longueurs émettrices des irradiateurs tubulaires (8) peuvent être changées, et que l'intensité de l'irradiation est modulable par variations de la puissance –HF donnée par un système électronique ballast connu en lui-même.

2. Dispositif selon la revendication 1, caractérisé en ce qu'on prévoit comme irradiateur (8) une lampe au Xénon.

3. Dispositif selon les revendications 1 et 2, caractérisé en ce que le domaine spectrale nécessaire à l'efficacité réalisée par apport d'une substance luminescente spéciale sur l'irradiateur (8).

4. Dispositif selon les revendications 1 à 3, caractérisé en ce que l'irradiation du sang dans le tube (7) s'établit entre 1 mW cm$^{-2}$ et 10 mW cm$^{-2}$ au moyen d'une variation unique ou combinée de l'intensité d'irradiation ainsi que de la longueur de la section du tube irradié de 1 à 10 cm ainsi que du débit du sang irradié de 20 à 80 gouttes/minute.

Fig. 1

Fig. 2